(19) Europäisches Patentamt
European Patent Office
Office européen des brevets

(11) Numéro de publication: **0 497 658 B1**

(12) **FASCICULE DE BREVET EUROPEEN**

(45) Date de publication du fascicule du brevet: **09.08.95**

(51) Int. Cl.$^6$: **C07D 403/12**, A61K 31/505

(21) Numéro de dépôt: **92400163.9**

(22) Date de dépôt: **22.01.92**

# (54) Utilisation de dérivés 1-H-azole-(omega-(4-(2-pyrimidinyl)-1-pipérazinyl)-alkyl) pour la préparation de médicaments destinés au traitement des troubles des fonctions cognitives.

(30) Priorité: **25.01.91 FR 9100861**

(43) Date de publication de la demande:
**05.08.92 Bulletin 92/32**

(45) Mention de la délivrance du brevet:
**09.08.95 Bulletin 95/32**

(84) Etats contractants désignés:
**AT BE CH DE DK FR GB GR IT LI LU NL PT SE**

(56) Documents cités:
**EP-A- 0 382 637**
**EP-A- 0 429 360**

**R. A. Glennon, Neuroscience and Behavioral Reviews, vol. 14, pp 35- 47 (1990)**

(73) Titulaire: **LABORATORIOS DEL DR. ESTEVE, S.A.**
**Av. Mare de Deu de Montserrat, 221**
**E-08026 Barcelona (ES)**

(72) Inventeur: **Constansa, Jordi Frigola**
**Av. Diagonal, 299 at.la**
**E-08013 Barcelone (ES)**
Inventeur: **Corominas, Juan Parés**
**Padilla, 349, 3o 3a**
**E-08025 Barcelone (ES)**

(74) Mandataire: **Ahner, Francis**
**CABINET REGIMBEAU**
**26, avenue Kléber**
**F-75116 Paris (FR)**

EP 0 497 658 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne l'utilisation des dérivés de 1H-azole-(ω-(4-(2-primidinyl)-1-pipérazinyl)-alkyl) ainsi que de leurs sels physiologiquement acceptables, pour la fabrication de médicaments destinés au traitement des troubles des fonctions cognitives, tels que la démence présénile, la démence sénile, les disfonctions de la mémoire, la détérioration de la conscience, et en particulier la maladie d'Alzheimer.

Alors que tous les anxiolithiques benzodiazépiniques se sont toujours révélés incapables de traiter les troubles mentaux affectant les fonctions cognitives, il a été constaté de façon totalement surprenante que certains dérivés de 1H-azole-(ω-(4-(2-primidinyl)-1-pipérazinyl)-alkyl) étaient très actifs pour traiter ces troubles particuliers du système nerveux central.

"L'état de la technique antérieure peut être complété par EP-A-0 382 637 et EP-A-0 429 360, décrivant l'application des dérivés 1H-azole-(ω-4)-(4-(2-pyrimidinyl)-1-pipérazinyl)-alkyl, respectivement comme tranquillisant et pour le traitement du syndrome d'abstinence".

Les composés préconisés dans le cadre de la présente invention répondent à la formule générale I

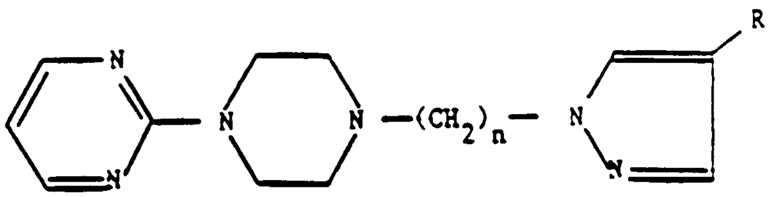

(I)

dans laquelle :

n peut avoir les valeurs 1 à 6, et

R représente un atome d'hydrogène, un halogène, un radical alkyle inférieur en $C_1$ à $C_4$, un radical heteroaryle, un radical sulfonique, un radical sulfamido N-substitué ou N-disubstitué,

un radical nitro, un radical hydroxy, un radical oxo, un radical alcoxy inférieur en $C_1$ à $C_4$, un radical cyano, un radical carboxylate d'alkyle inférieur en $C_1$ à $C_4$, un radical aryle ou aryle substitué, un radical amino ou amino substitué, de formule

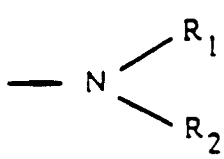

dans laquelle

$R_1$ et $R_2$, identiques ou différents, représentent un atome d'hydrogène, un radical alkyle, un radical aryle, un radical alkylcarboxy, un radical arylcarboxy, un radical alkylsulfonyle ou un radical arylsulfonyle, les fragments alkyle de ces radicaux contenant de 1 à 4 atomes de carbone.

Ces dérivés de formule générale I peuvent être préparés, conformément à l'invention, selon l'une quelconque des méthodes suivantes.

Méthode A

Par réaction d'un composé de formule générale II

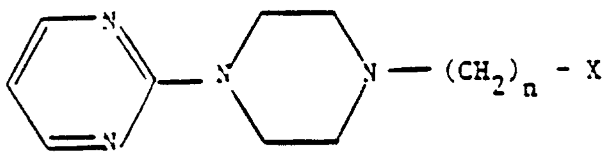

(II)

dans laquelle

X représente un atome d'halogène ou un groupe partant choisi parmi le tosyloxy ou le mésyloxy, avec un composé de formule générale III

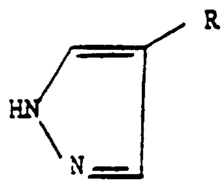

(III)

dans laquelle

R a les significations mentionnées précédemment.

La réaction s'effectue en présence d'un solvant adéquat, par exemple le diméthylsulfoxyde, la diméthylformamide, un alcool, un hydrocarbure, aromatique ou non, un éther, tel le dioxanne ou l'éther diphénylique, ou un mélange de ces solvants. Cette réaction est avantageusement conduite en présence d'une base telle les hydroxydes, les carbonates ou les bicarbonates des métaux alcalins, ou bien d'un mélange de ces bases. Les températures les plus adéquates varient entre la température ambiante et la température de reflux du solvant, et le temps réactionnel est compris entre 1 heure et 24 heures.

Méthode B

Par réduction d'un composé de formule générale I, dans laquelle

R représente un groupement nitro.

Parmi les nombreux agents réducteurs susceptibles d'être utilisés pour réduire un groupe nitro jusqu'en un groupe amino, on peut citer les suivants : l'hydrogénation catalytique, en utilisant comme catalyseurs le nickel, le palladium ou le platine, l'amalgame de zinc avec de l'acide chlorhydrique, les borohydrures de métaux alcalins, etc.

La réaction s'effectue au sein d'un alcool, tel le méthanol, l'éthanol ou l'un quelconque des propanols ou des butanols, ou bien un mélange d'un alcool avec de l'eau. Les températures les plus appropriées sont comprises entre -10 °C et celle de reflux du solvant, et le temps de réaction est compris entre 1 heure et 24 heures.

Méthode C

Par acylation d'un composé de formule générale I dans laquelle

R représente un groupement amino, avec un halogénure d'acide ou un anhydride.

La réaction s'effectue sans solvant ou en présence d'un solvant adéquat, tel un hydrocarbure, une cétone ou un éther, et en présence d'une base, comme la pyridine ou les trialkylamines. Les températures les plus appropriées varient entre -10 °C et la température d'ébullition du solvant et le temps réactionnel est compris entre 1 heure et 24 heures.

Méthode D

Par réduction alkylative d'un composé de formule générale I, dans laquelle

R représente un groupement nitro, cette réduction alkylative étant réalisée avec un borohydrure de métal alcalin en présence de chlorure de nickel II et d'un composé qui possède un groupement cétone ou aldéhyde. Cette réaction s'effectue au sein d'un alcool ou d'un mélange d'alcool et d'eau. Les températures les plus convenables varient entre -15 ° C et celle de reflux du solvant, et le temps réactionnel est compris entre quelques minutes et 24 heures.

Méthode E

Par réaction d'un composé de formule générale IV

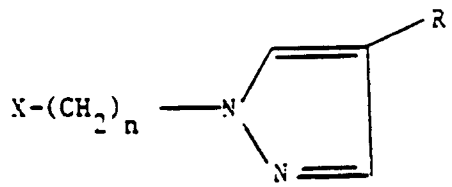

(IV)

dans laquelle

X et n on les significations mentionnées précédemment, avec un composé de formule générale V

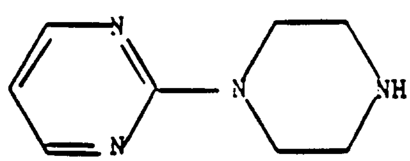

(V)

Les exemples suivants illustrent la préparation de quelques dérivés entrant dans le cadre de la présente invention. On décrira également quelques formes d'emploi.

Méthode A

Exemple 1

Préparation de 1H-pyrazole-1-(4-(4-2-pyrimidinyl)-1-pipérazinyl)-butyl)

On chauffe à reflux pendant 14 heures un mélange de 4g (13,3 mmoles) de 2-pyrimidine-1-(4-bromobutyl)-4-pipérazine, 1,02g (15 mmoles) de pyrazole et 2,76 g (20 mmoles) de carbonate de potassium, dans 50ml de diméthylformamide. On évapore sous vide, on ajoute du chloroforme, on lave à l'eau, on sèche sur du sulfate de sodium, on évapore sous vide et on obtient 3,5g d'une huile qui est le 1H-pyrazole-1-(4-(4-(2-pyrimidinyl)-1-pipérazinyl)-butyl).

Les composés identifiés par les exemples 1 à 9 sont obtenus par la même procédure et les données pour leur identification sont exposées dans le tableau I.

Méthode B

Exemple 10

Préparation de 1H-pyrazole-4-amino-1-(4-(4-(2-pyrimidinyl)-1-pipérazinyl)-butyl)

On ajoute 10,2g (43,2 mmoles) de chlorure de nickel II hexahydrate à une solution de 7,2g (21 mmoles) de 1H-pyrazole-4-nitro-1-(4-(4-(2-pyrimidinyl)-1-pipérazinyl)-butyl), exemple n°7, dans 60ml d'éthanol, sous forte agitation. On refroidit avec un bain de glace et on ajoute lentement 10,2g (81 mmoles) de borohydrure de sodium. On laisse sous agitation pendant 1 heure et après 1 heure à la température ambiante, on additionne de l'eau, on évapore sous vide, on acidifie avec de l'acide chlorhydrique concentré, on filtre, on basifie avec de l'ammoniaque et on extrait à l'éther éthylique. On obtient ainsi 4,4g de 1H-pyrazole-4-amino-1-(4-(4-(2-pyrimidinyl)-1-pipérazinyl)-butyl) sous une forme liquide.

Les données spectroscopiques pour son identification sont données dans le tableau II.

Méthode C

Exemple 11

Préparation de 1H-pyrazole-4-méthylsulfonamido-1-(4-(4-(2-pyrimidinyl)-1-pipérazinyl)-butyl)

On ajoute lentement 1,8g (16 mmoles) de chlorure de méthanesulfonyle sur une solution refroidie de 4,4g (14,6 mmoles) de 1H-pyrazole-4-amino-1-(4-(4-(2-pyrimidinyl)-1-pipérazinyl)-butyl), exemple 16, dans 30ml de pyridine. On laisse pendant une heure à 0°C, on abandonne à température ambiante pendant 4 heures, on verse sur de l'eau glacée, on extrait du chloroforme, et on obtient 3,7g de 1H-pyrazole-4-méthylsulfonamido-1-(4-(4-(2-pyrimidinyl)-1-pipérazinyl)-butyl), qu'on peut recristalliser dans de l'éther éthylique, avec un point de fusion de 132°C.

Les composés identifiés par les exemples 12 et 13 sont obtenus par la même méthode et les données pour leur identification sont exposées dans le tableau II.

Méthode D

Exemple 14

Préparation de 1H-pyrazole-4-(2-butyl)amino-1-(4-(4-(2-pyrimidinyl)-1-pipérazinyl)-butyl)

On ajoute 0,9g (24 mmoles) de borohydrure de sodium à une suspension de 2,8g (12 mmoles) de dichlorure de nickel hexahydrate, dans une solution de 2 g (6 mmoles) de 1H-pyrazole-4-nitro-1-(4-(4-(2-pyrimidinyl)-1-pipérazinyl)-butyl), exemple 7, et 10 ml de méthyléthylcétone dans 50ml d'éthanol, refroidie à 0°C. On maintient cette température pendant 30 minutes, on laisse monter jusqu'à température ambiante, on poursuit l'agitation pendant 2 heures, on évapore sous vide, on reprend avec de l'acétate d'éthyle et on obtient 1,22g de 1H-pyrazole-4-(2-butyl)amino-1-(4-(4-(2-pyrimidinyl)-1-pipérazinyl)-butyl), sous une forme liquide.

Les données spectroscopiques de ce produit sont indiquées dans le tableau II.

TABLEAU I

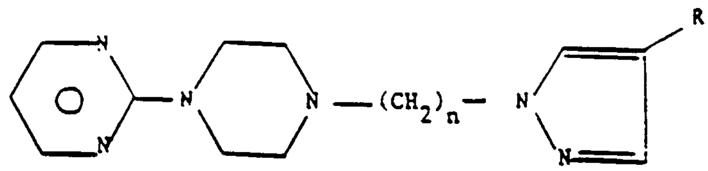

| Ex. | R | n | IR $cm^{-1}$ | 1H RMN, δ, $CDCl_3$, J=Hz |
|---|---|---|---|---|
| 1 | H | 4 | 2942, 2815, 1586, 1547, 983 | 1.50(m,2H); 1.90(m,2H); 2.40(m,6H); 3.80(m,4H); 4.12(t,2H,J=6,9); 6.20 (t,1H,J=1,6); 6.40(t,1H, J=4.7); 7.42(dd,2H,J=4.7; J'=1,6); 8.25(d.2H,J=4.7) |

TABLEAU I (suite)

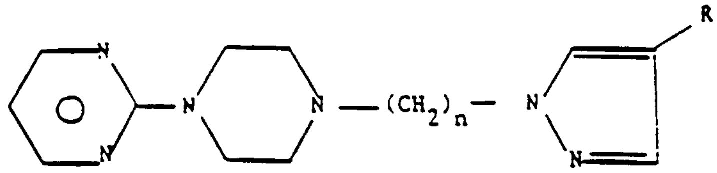

| | | | | |
|---|---|---|---|---|
| 2 | Me | 4 | 1590. 1550, 1500. 1360 1260. 980 | 1.52(m.2H: 1.95(m.2H): 2.05(s.3H): 2.37(m.6H): 3.81(m.4H); 4.05(t.2H.J= 6.8); 6.41(t,1H:J=4.7)7.13 (S,1H); 7.27(s,1H); 7.27 (s,1H; 8.25(d.2H,J=4.7) |
| 3 | $NO_2$ | 4 | 1584. 1524, 1480. 1444, 1406, 1359, 1305, 819 | 1.5(m.2H); 1.93(m.2H); 2.38(m,6H); 3.76(m.4H); 4.15(t,2H,J=6.7); 6.42 (t,1H,J=4.7); 8.01(s,1H) 8.12(s.1H); 8.24(d,2H,J=4.7) |
| 4 | Cl | 4 | 2843, 1586, 1547, 1358, 983 | 1.52(m,2H); 1.90(m,2H); 2.43(m,6H); 3.80(m.4H); 4.0(t,2H,J=6.8); 6.44(t,1H, J=4.7); 7.35(s,1H) 7.39 (s.1H): 8.25(d.2H.J=4.7) |

TABLEAU I (suite)

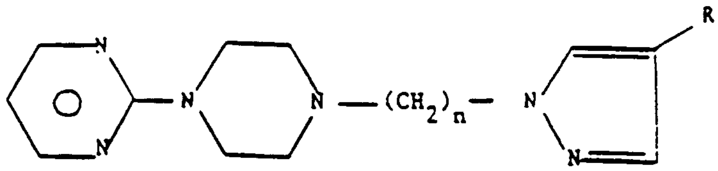

| | | | | |
|---|---|---|---|---|
| 5 | Et-OOC- | 4 | 1715, 1586, 1222, 983 | 1.34(t.3H.J=7.1); 1.54 (m.2H); 1.90(m.2H); 2.46 (m.6H); 3.81(m.4H); 4.25 (m.4H); 6.47(t.1H.J=4.7); 7.90(s.2H); 8.29(d.2H.J=4.7) |
| 6 | Br | 4 | 1586, 1547, 1360, 984 | 1.52(m.2H); 1.89(m.2H); 2.44(m.6H); 3.62(m.4H); 4.11(t,2H,J=6.7) : 6.46 (t,1H,J=4.6); 7.42(s,1H); 7.45(s,1H); 8.29(d,2H,J=4.6) |
| 7 | C≡N | 4 | 3076, 2231 1587, 1551 1258, 982 | 1.54(m,2H); 1.96(m,2H); 2.40(m.6H); 3.81(m.4H); 4.20(t,2H,J=6.9) : 6.48 (t,1H,J=4.7); 7.80(s,1H); 7.83(s,1H); 8.29(d.2H.J=4.7) |

TABLEAU I (suite)

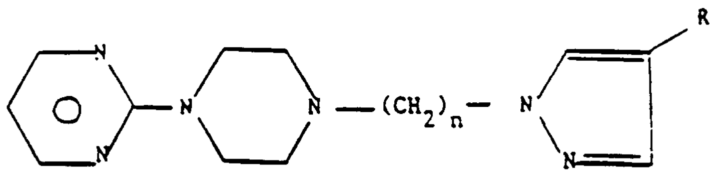

| | | | | |
|---|---|---|---|---|
| 8 | F | 4 | 2944, 1584 1546, 1507, 1359, 1260 983 | 1.45(m,2H); 1.96(m.2H): 2.36(m.6H); 3.77(m.4H); 4.0(t,2H,J=6.9); 6.47 (t,1H,J=4.7); 7.27(d.1H, J=4.8); 8.29(d,2H,J=4.8) |
| 9 | Me-0- | 4 | 2940, 1585, 1547, 1470, 1359, 1122, 983 | 1.54(m,2H); 1.89(m.2H); 2.42(m.6H); 3.77(m.7H); 4.06(m,2H); 6.42(t,1H, J=4.7); 7.02(s,1H)7.26 (s,1H); 8.25(d,2H,J=4.6) |

TABLEAU II

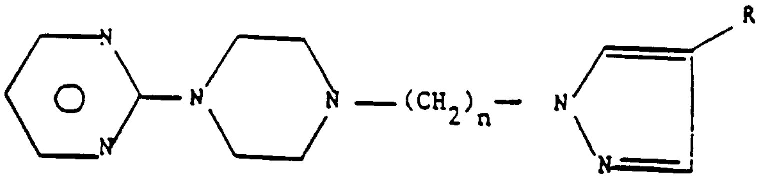

| Ex. | R | P.F. | n | IR | 1H RMN, δ, $CDCl_3$, J=Hz |
|---|---|---|---|---|---|
| 10 | $H_2N$- | huile | 4 | 1586, 1548, 1360, 984 | 1.50(m,2H); 1.85(m,2H) 2.43(m,6H); 3.4 (élargie 2H); 3.8(m,6H); 4.0 (t,24,J=6.4); 6.46(t,1H, J=4.7); 6.98(s,1H); 7.10 (s,1H); 8.27(d,2H,J=4.7) |

TABLEAU II (suite)

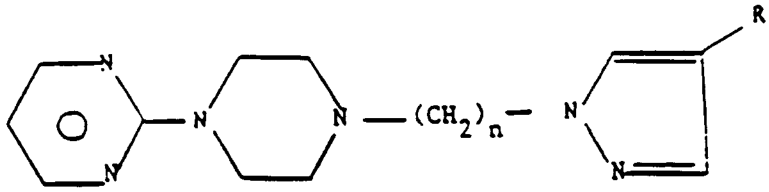

| | | | | | |
|---|---|---|---|---|---|
| 11 | Me-$SO_2$-NH- | 132°C | 4 | 1582, 1482, 1360, 1150, 983 | 1.58(m,2H); 1.93(m,2H) 2.45(m,6H); 2.94(s,3H); 3.8(m,4H);4.11(t,2H,J =6.9); 6.45(t,1H,J=4.7); 7.4(s,1H); 7.5(s,1H) 8.28(d,2H,J=4.7) |

TABLEAU II (suite)

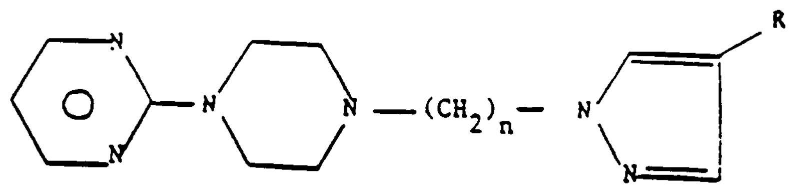

| | | | | | |
|---|---|---|---|---|---|
| 12 | Ph-CO-NH- | 134-6°C | 4 | 1646, 1586, 1542. 1369 | 1.55(m,2H); 1.79(s,3H); 1.88(m,2H); 2.42(m,6H); 3.80(m,4H); 4.13(t,2H, J=6.8); 6.51(t,1H,J=4.7); 7.49(m,4H); 7.83 (m,2H); 8.0(s,1H); 8.11 (s.1H); 8.28(d,2H,J=4.7) |

TABLEAU II (suite)

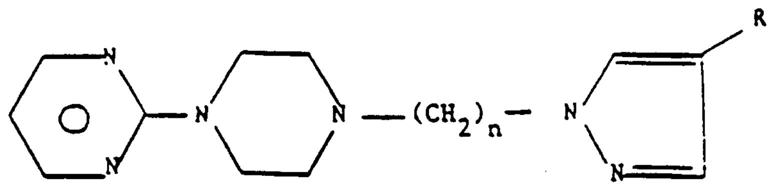

| | | | | | |
|---|---|---|---|---|---|
| 13 | Me-CO-NH- | 80-2°C | 4 | 1650, 1586, 1454, 1364, 1261, 983 | 1.50(m.2H); 1.88(m.2H); ;2.11(s,3H); 2.43(m,6H) ;3.79(m,4H); 4.08(t,2H, J=6.8); 6.47(t,1H,J=4.7) ; 7.36(s,1H)7.93(s,1H); 8.28(d,2H,J=4.6); 9.25 (s,1H) |
| 14 | 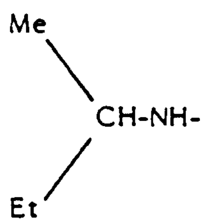 | Huile | | 2960, 1585, 1547, 1359, 1260 983 | 1.00(t,3H,J=7.0); 1.19 (d,3H,J-6.3); 1.6(m,4H); 1.90(m,2H); 2.50(m,6H) ; 3.0(m,3H); 3.9(m,4H); 4.1(t,2H,J=6.8); 6.52 (t,1H,J=4.7); 6.99(s.1H); 7.17(s,1H); 3.37(d,2H, J=4.7) |

TABLEAU II (suite)

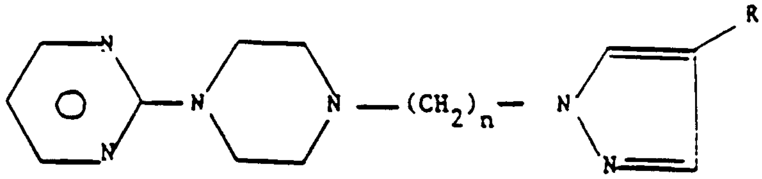

| | | | | | |
|---|---|---|---|---|---|
| 15 | 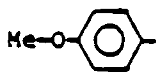 | 79-82°C | 4 | 2390, 1589, 1545, 1495, 1360, 1247, 983, 835, 799 | 1.62(m,2H); 1.88(m,2H); 2,45(m,6H); 3.81(m,7H); 4.16(t,2H,J=6,8); 6.46 (t,1H,J=4,7); 6.9 (d,2H,J=4,4); 7.4 (d,2H,J=4,4); 7.55 (s,1H); 7.7(s,1H); 8,28(d,2H,J=2,4) |
| 16 | 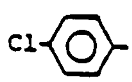 | 108-110°C | 4 | 2946, 1586, 1549, 1485, 1395, 1257, 982, 951, 830 | 1.6(m,2H); 1.9(m,2H); 2.46(m,6H); 3.8(m,4H); 4.16(t,2H,J=6,8); 6.4 (t,1H,J=4,7); 7.36 (d,4H,J=1,3); 7.7 (d,2H,J=6,2); 8.28 (d,2H,J=2,3) |
| 17 | 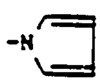 | Huile | 4 | 2943, 1586, 1487, 1359, 1260, 984, 726, | 1.55(m,2H); 1.80(m,2H); 2.45(m,6H); 3.81 (t,4H,J=5); 4.12 (t,2H,J=7); 6.25 (2H,t,J=2); 6.44 (1H,t,J=4,7); 6.84 (m,2H); 7.5(d,2H,J=5); 8.27(d,2H,J=4,7) |
| 18 | 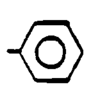 | 39-42°C | 4 | 2942, 1585, 1493, 1446, 1359, 1258, 983, 760 | 1.6(m,2H); 1.9(m,2H); 2.5(m,4H); 3.8(m,6H); 4.2(t,2H,J=6,8); 6.7 (t,1H,J=4,7); 7.2-7.7 (abs. compl. 5H); 8.0 (s,1H); 8.2(s,1H); 8.4 (d,2H,J=2,3) |

TABLEAU II (suite)

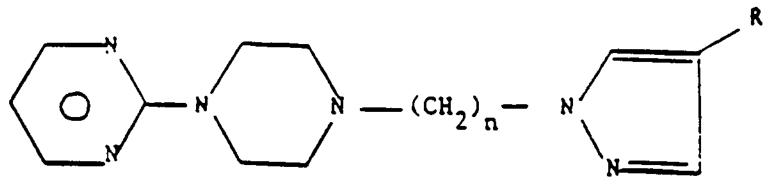

| | | | | | |
|---|---|---|---|---|---|
| 19 | 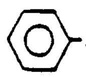-$SO_2$-NH- | 92-95°C | 4 | 2931, 1584, 1548, 1490, 1358, 1167, 983 | 1.45(m,2H); 1.85(m,2H); 2.40(m,6H); 3.80(m,4H); 4.0(t,2H,J=6,7); 6.47 (t,1H,J=4,6); 7.0 (s,1H); 7.5(m,6H); 8.3 (d,2H,J=4,6) |
| 20 | Me-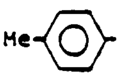-$SO_2$-NH | 108-110°C | 4 | 2943, 1585, 1548, 1446, 1360, 1161, 984 | 1.5(m,2H); 1.85(m,2H); 2.28(m,9H); 3.8(m,4H); 4.0(m,2H); 6.45 t,1H,J=4,7); 7-7.65 (m,6H); 8.27 (d,2H,J=4,7) |
| 21 | n-Bu-$SO_2$-NH- | Huile | 4 | 2941, 1586, 1548, 1448, 1360, 1146, 984, 755 | 0.91(t,3H,J=6,8); 1.45 (m,4H); 1.85(m,4H); 2.40(m,6H); 3.0(m,2H); 3.80(m,4H); 4.11 (t,2H,J=6,5); 6.5 (t,1H,J=4,7); 7.4 (m,2H); 7.5(s,1H); 8.3 (d,2H,J=4,7) |
| 22 | n-Pr-$SO_2$-NH- | Huile | 4 | 2940, 1586, 1548, 1447, 1360, 1146, 984, 755 | 1.0(t,3H,J=7,1); 1.55 (m,2H); 1.9(m,4H); 2.45(m,6H); 3.0 (t,2H,J=7,4); 3.8 (m,4H); 4.1 (t,2H,J=6,4); 6.46 (t,1H,J=4,7); 7.35 (m,2H); 7.5(s,1H); 8.3 (d,2H,J=4,7) |

TABLEAU II (suite)

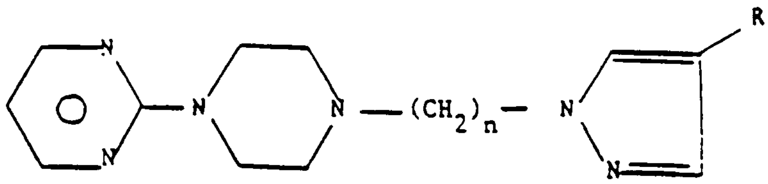

| | | | | | |
|---|---|---|---|---|---|
| 23 | $Et-SO_2-NH-$ | Huile | 4 | 2943, 1586, 1548, 1447, 1360, 1146, 984, 754 | 1.36(m,5H); 1.9(m,2H); 2.45(m,6H); 3.0(m,2H); 3.6(m,4H); 4.1 (t,2H,J=6,4); 6.45 (t,1H,J=4,7); 7.39 (s,1H); 7.51(s,1H); 8.3(d,2H,J=4,7) |
| 24 | $-SO_2-N-Me_2$ | 100-102°C | 4 | 3135, 2943, 1586, 1512, 1357, 1328, 1156, 982, 728 | 1.6(m,2H); 1.9(m,2H); 2.3-2.7(abs. compl. 13H); 3.8(m,4H); 4.2 (t,2H,J=6,8); 6.4 (t,1H,J=4,7); 7.75 (d,1H,J=4,4); 8.28 (d,2H,J=2,4) |
| 25 | $-SO_3-H$ | 230-235°C (desc.) | 4 | 3330, 1590, 1556, 1449, 1220, 1178, 1049, 971, 656 | 1.95(m,2H); 3.3(m,6H); 4.0(s,5H); 4.27 (t,2H,J=6,1); 6.8 (t,1H,J=4,8); 7.8 (s,1H); 8.0(s,1H); 8.43 (d,2H,J=2,4) |

TABLEAU II (suite)

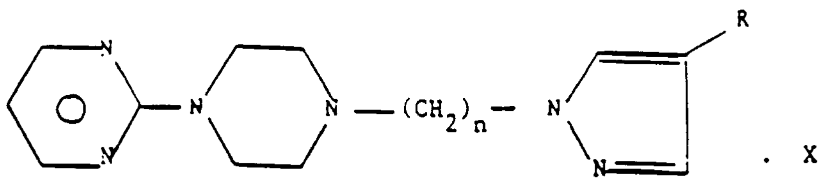

| Ex. | R | X | P.F. | n | IR $cm^{-1}$ |
|---|---|---|---|---|---|
| 26 | Cl | 1 HCl | 156-158°C | 4 | 3490, 1592, 1556, 1481, 1438, 1386, 970 |
| 27 | Cl | 2 $HCl.H_2O$ | 194-197.5°C | 4 | 3429, 2688, 1636, 1620, 1346, 1218, 971 |

Activité pour l'amélioration de la cognition

On démontre pour les composés de la formule I précitée l'existence d'une activité sur l'amélioration de la cognition de la manière suivante :

On étudie l'influence des produits à tester sur le processus d'accoutumance des souris dans le test de la boîte claire/obscure décrit par J.M. Barnes et col. (Pharmacol. Biochem. Behav., 1990, 35, 955-962). On étudie d'un côté l'effet sur l'apprentissage (la vitesse d'accoutumance) et d'un autre côté la capacité de blocage des effets contraires produits par l'escopolamine.

On place la souris dans la zone claire d'une boîte divisée en deux compartiments, un très illuminé, boîte claire, et l'autre peu illuminé, boîte obscure.

1) On compte le nombre de fois que la souris se dresse sur les pattes arrières dans chaque compartiment pendant 5 minutes. (voir colonne 1 du tableau ci-après)

2) L'activité dans chaque compartiment est donnée par le comptage du nombre de croisements par les carreaux qui constituent les divisions de chaque compartiment. (voir colonne 2 du tableau ci-après)

3) On mesure le temps passé dans la boîte obscure pendant les 5 minutes du comptage. (voir colonne 3 du tableau ci-après)

4) On détermine la latence initiale, c'est-à-dire le temps passé depuis que l'on place l'animal dans la boîte claire, au début de l'essai, jusqu'à sort entrée dans la boîte obscure. (voir colonne 4 du tableau ci-après)

On administre aux animaux témoins deux traitements par jour à l'aide du seul excipient. On administre aux animaux traités avec le composé à expérimenter deux doses par jour de 0,01 mg/kg, ip, de ce produit. La même opération est répétée journellement pendant 3 jours. Les animaux apprennent à rester plus de temps dans la boîte obscure et à s'y rendre plus rapidement.

Au quatrième jour on administre de l'escopolamine (2 x 0,25 mg/kg, ip). Avec ce traitement les animaux du groupe témoin "oublient" le comportement appris consistant à rester plus de temps dans la boîte obscure.

Le traitement avec un produit qui améliore la connaissance fait que :

1) le comportement appris est amélioré, l'apprentissage est fait plus rapidement et le temps de résidence dans la boîte obscure augmente.

2) La réversion de l'apprentissage produite par l'escopolamine est totalement bloquée.

Les données obtenues sont résumées dans le tableau III. Elles démontrent, sur la base des résultats obtenus par exemple avec le composé de l'exemple 27, une activité d'amélioration de la cognition, étant donné que ce composé améliore le processus de l'apprentissage et bloque les effets de l'escopolamine. Le piracétam testé sous les mêmes conditions n'a aucune activité.

TABLEAU III

INFLUENCE DU COMPOSE DE L'EXEMPLE 27 (0.01 MG/KG, IP, DEUX FOIS PAR JOUR) SUR LE PROCESSUS D'ACCOUTUMANCE CHEZ LA SOURIS DETERMINE PAR LE TEST DE LA BOITE CLAIRE/OBSCURE.

| JOUR DE TRAITEMENT | REDRESSEMENTS EN 5 MIN. | | | | ACTIVITE EN 5 MIN | | | | TEMPS EN BOITE | | LATENCE | |
|---|---|---|---|---|---|---|---|---|---|---|---|---|
| | TEMOIN C | TEMOIN O | EXEMPLE 27 C | EXEMPLE 27 O | TEMOIN C | TEMOIN O | EXEMPLE 27 C | EXEMPLE 27 O | TEMOIN O | EXEMPLE 27 O | TEMOIN C - O | EXEMPLE 27 C - O |
| 1 | 22 | 70 | 22 | 78 | 35 | 82 | 38 | 85 | 59 | 60 | 9 | 9 |
| 2 | 23 | 71 | 8 * | 125 * | 36 | 83 | 12 * | 138 * | 60 | 87 * | 7 | 2 * |
| 3 | 19 | 72 | 8 * | 130 * | 30 | 84 | 8 * | 145 * | 60 | 80 * | 7 | 0.5 * |
| 4 (*) | 40 + | 20 + | 7 * o | 125 * o | 55 + | 30 + | 9 * o | 148 * o | 20 + | 80 * o | 24 + | 1 * o |

(*) 2 traitements préalables avec de l'escopolamine (0.25 mg/kg, ip)
* $p < 0.001$ (amélioration de l'apprentissage, comparé avec le jour 1)
\+ $p < 0.001$ (réversion du procès d'accoutumance produit par l'escopolamine)
o $p < 0.001$ (inhibition de l'effet de l'escopolamine)
C - O : Claire - obscure

Activité anti-dépressive

De surcroît, on a démontré l'existence d'une activité antidépressive pour l'exemple 27 en utilisant le test de comportement désespéré chez la souris décrit par R.D. Porsolt et col. (Arch. Int. Pharmacodyn., 1977, 229, 327-336). Les animaux sont placés, pendant 6 minutes, dans un cylindre contenant de l'eau, dont ils ne peuvent pas s'échapper. On mesure, en groupes de 10 souris par dose testée, la durée de l'immobilité entre les minutes 2 et 5.

Le produit étudié est administré par voie ip, 1 heure avant le test. Dans ce test, on explique l'immobilité des animaux comme produite par leur état dépressif ("désespéré") conséquence d'être mis face à une situation adverse et insoluble dans un environnement hostile comme l'eau. Les antidépressifs réduisent cette immobilité. Dans notre essai on a utilisé l'imipramine (30 mg/kg, ip) comme produit de référence.

Les résultats démontrent que le composé 27 a une activité anti-dépressive puis qu'il réduit significativement le temps d'immobilité du groupe témoin.

| Produit | Doses (mg/kg,ip ) | Temps d'immobilité (secondes) |
|---|---|---|
| Témoin | - | 87 |
| Exemple 27 | 1 | 58 (p 0.05) |
| Imipramine | 30 | 32 (p 0.05) |

Les dérivés de formule générale I selon l'invention sont donc utiles comme substances actives de médicaments destinés au traitement de troubles associés à la cognition, tels que la démence sénile, les disfonctions de la mémoire, la détérioration de la conscience, etc., ainsi que les états dépressifs.

En thérapeutique humaine, la dose administrée est bien sûr fonction de la gravité des troubles particuliers du système nerveux central.

Elle sera généralement comprise entre environ 5 et environ 100 mg/jour.

Les dérivés de l'inention seront, par exemple, administrés sous forme de comprimés, de solutions ou de suspensions, ou bien de gélules.

On indiquera ci-après, à titre d'exemples, deux formes galéniques particulières.

| **Exemple de formule par comprimé** | |
|---|---|
| Composé 27 | 5 mg |
| Lactose | 60 mg |
| Cellulose microcristalline | 25 mg |
| Povidone | 5 mg |
| Amidon prégélatinisé | 3 mg |
| Dioxyde de silice colloïdale | 1 mg |
| Stéarate de magnésium | 1 mg |
| | Poids comprimé 100 mg |

| **Exemple de formule par gélule** | |
|---|---|
| Composé 27 | 10 mg |
| Glycérine polyoxyéthylénée | 135mg |
| Béhénate de glycérine | 5 mg |
| | 150 mg |
| Excipient : gélatine molle q.s. | |

## Revendications

**1.** Utilisation des dérivés de formule générale I

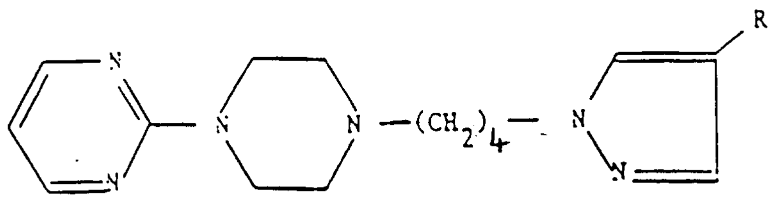

(I)

dans laquelle :
R représente un atome d'halogène, en particulier de chlore, et leurs sels thérapeutiquement acceptables,
pour la fabrication de médicaments destinés au traitement des troubles des fonctions cognitives, tels que la démence présénile, la démence sénile, les disfonctions de la mémoire, la détérioration de la conscience et la maladie d'Alzheimer.

**2.** Utilisation selon la revendication 1, caractérisée en ce que le dérivé de formule générale I est choisi parmi :
1. 1H-pyrazole-4-chloro-1-(4-(4-(2-pyrimidinyl)-1-pipérazinyl)-butyl),
2. 1H-pyrazole-4-bromo-1-(4-(4-(2-pyrimidinyl)-1-pipérazinyl)-butyl),
3. 1H-pyrazole-4-fluoro-1-(4-(4-(2-pyrimidinyl)-1-pipérazinyl)-butyl),
4. 1H-pyrazole-4-chloro-1-(4-(4-(2-pyrimidinyl)-1-pipérazinyl)-butyl)hydrochlorure,
5. 1H-pyrazole-4-chloro-1-(4-(4-(2-pyrimidinyl)-1-pipérazinyl)-butyl)dihydrochlorure.

## Claims

**1.** Use of the derivatives of general formula I

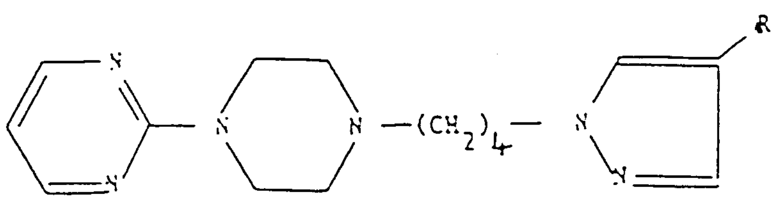

(I)

in which:
R represents a halogen atom, especially a chlorine atom, and their therapeutically acceptable salts, for the manufacture of medicinal products intended for the treatment of disorders of cognitive function, such as presenile dementia, senile dementia, memory dysfunctions, deterioration of awareness and Alzheimer's disease.

**2.** Use according to Claim 1, characterised in that the derivative of general formula I is chosen from:
1. 4-chloro-1-{4-[4-(2-pyrimidinyl)-1-piperazinyl]-butyl}-1H-pyrazole,
2. 4-bromo-1-{4-[4-(2-pyrimidinyl)-1-piperazinyl]-butyl}-1H-pyrazole,
3. 4-fluoro-1-{4-[4-(2-pyrimidinyl)-1-piperazinyl]-butyl}-1H-pyrazole,
4. 4-chloro-1-{4-[4-(2-pyrimidinyl)-1-piperazinyl]-butyl}-1H-pyrazole hydrochloride,
5. 4-chloro-1-{4-[4-(2-pyrimidinyl)-1-piperazinyl]-butyl}-1H-pyrazole dihydrochloride.

**Patentansprüche**

1. Verwendung von Derivaten der allgemeinen Formel (I)

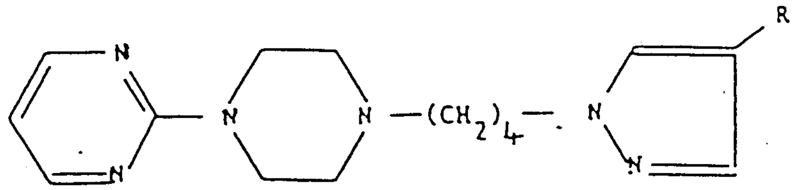

(I)

worin R ein Halogenatom, insbesondere ein Chloratom, bedeutet, und ihrer therapeutisch akzeptablen Salze für die Herstellung von Arzneimitteln zur Behandlung von kognitiven Funktionsstörungen wieder präsenilen Demenz, der senilen Demenz, von Gedächtnisstörungen, Bewußtseinsstörungen und der Alzheimer-Krankheit.

2. Verwendung nach Anspruch 1, dadurch gekennzeichnet, daß das Derivat der allgemeinen Formel (I) ausgewählt wird aus der Gruppe:
   1. 1H-Pyrazol-4-chloro-1-(4-(4-(2-pyrimidinyl)-1-piperazinyl)-butyl),
   2. 1H-Pyrazol-4-bromo-1-(4-(4-(2-pyrimidinyl)-1-piperazinyl)-butyl),
   3. 1H-Pyrazol-4-fluoro-1-(4-(4-(2-pyrimidinyl)-1-piperazinyl)-butyl),
   4. 1H-Pyrazol-4-chloro-1-(4-(4-(2-pyrimidinyl)-1-piperazinyl)-butyl) hydrochlorid,
   5. 1H-Pyrazol-4-chloro-1-(4-(4-(2-pyrimidinyl)-1-piperazinyl)-butyl) dihydrochlorid.